(19) Europäisches Patentamt
European Patent Office
Office européen des brevets

(11) **EP 3 591 061 A1**

(12) **EUROPEAN PATENT APPLICATION**
published in accordance with Art. 153(4) EPC

(43) Date of publication:
**08.01.2020 Bulletin 2020/02**

(51) Int Cl.:
**C12P 1/02** *(2006.01)*    **C12P 21/00** *(2006.01)*
**C12P 21/02** *(2006.01)*

(21) Application number: **18760802.1**

(22) Date of filing: **28.02.2018**

(86) International application number:
**PCT/JP2018/007420**

(87) International publication number:
**WO 2018/159652 (07.09.2018 Gazette 2018/36)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD TN**

(30) Priority: **01.03.2017   JP 2017038078**

(71) Applicant: **Sanyo Chemical Industries, Ltd.
Kyoto 605-0995 (JP)**

(72) Inventors:
• **UEDA, Masumi
Kyoto-shi
Kyoto 605-0995 (JP)**

• **NAKANISHI, Mutsumi
Kyoto-shi
Kyoto 605-0995 (JP)**
• **CHIBA, Yasunori
Tsukuba-shi
Ibaraki 305-8568 (JP)**
• **YOKO-O, Takehiko
Tsukuba-shi
Ibaraki 305-8568 (JP)**

(74) Representative: **Mewburn Ellis LLP
City Tower
40 Basinghall Street
London EC2V 5DE (GB)**

(54) **PRODUCTION METHOD FOR USEFUL SUBSTANCE**

(57)    The present invention provides a method of producing a useful substance, including secreting and producing a useful substance in broth by yeast contained in the broth, the yeast being a *Saccharomyces* strain, the broth containing a compound (A) represented by a formula (1), the compound (A) having an HLB value of 0.1 to 16 and a number average molecular weight of 200 to 30000, the weight percentage of the compound (A) based on the weight of the broth being 0.0001 to 10 wt%:

$$HO\text{-}(A^1O)m^1\text{-}(A^2O)m^2\text{-}(A^3O)m^3\text{-}H \qquad (1)$$

wherein $A^1O$, $A^2O$, and $A^3O$ each independently represent a C2-C4 oxyalkylene group; $A^1O$ and $A^2O$ have different structural formulas, $A^2O$ and $A^3O$ have different structural formulas; and $m^1$, $m^2$, and $m^3$ each respectively represent the average number of moles of $A^1O$, $A^2O$, and $A^3O$ added, and are each independently a number in the range of 1 to 600.

**EP 3 591 061 A1**

SEQUENCE LISTING

<110> SANYO CHEMICAL INDUSTRIES, LTD.

<120> Method for producing a useful substance

<130> 566-PCT

<150> JP2017-38078
<151> 2017-03-01

<160> 1

<170> PatentIn version 3.5

<210> 1
<211> 783
<212> DNA
<213> gaussia princeps

<400> 1

```
gaattcatga gatttccatc cattttcaca gcagtgctat ttgcggcatc atcagcttta       60

gcagccccag ttaataccac tacagaagat gaaactgctc aaattcctgc agaagcggtg      120

attggttact tggacttaga aggcgatttt gatgttgctg ttttgccgtt ctctaatagc      180

actaataatg ggctgttgtt cataaacact acaatcgcct ctatagcagc caaagaagaa      240

ggtgtatcct tggataaacg tgaggctgag aaaccaaccg aaaacaacga ggactttaac      300

attgtagcag ttgctagtaa ctttgcaacg accgatttgg acgcagatag aggcaaactt      360

cctggtaaga agttaccctt ggaagtccta aaagagatgg aggctaatgc gagaaaagct      420

ggctgtacta ggggatgctt gatttgcctt tcacacatca aatgtacgcc gaaaatgaag      480

aaattcatac caggtagatg ccatacctat gaaggtgaca aggaaagtgc tcaaggtgga      540

attggggaag ccatagtcga tattcccgaa atacctggtt ttaaggatct ggaacctatg      600

gagcagttca ttgctcaagt cgatctttgc gttgattgta ctacaggctg tttaaagggt      660

ttagccaatg tacagtgttc ggacctattg aagaaatggt taccacaaag atgtgccaca      720

tttgcttcta agatccaagg tcaagtggac aaaatcaaag gtgctggagg agattaagtc      780

gac                                                                    783
```

**Description**

TECHNICAL FIELD

The present invention relates to a method of producing a useful substance.

BACKGROUND ART

Yeast has been widely used to produce useful substances such as amino acids and proteins. Particularly in recent years, a technique has been known in which proteins are efficiently produced by yeast transformed by introducing a medically and/or industrially useful protein gene thereinto.
Use of *Escherichia coli* as a bacterium for production of useful substances is limited to production of proteins without sugar moieties because a glycan synthetase is absent in *Escherichia coli.* In contrast, use of yeast for production of useful substances can produce proteins with sugar moieties attached because proteins expressed in the yeast are secreted out of the bacterium after the proteins are modified by post-translational modification such as glycosylation by a glycan synthetase (Non-Patent Literature 1).
Yet, the protein production method using yeast has a lower protein yield than the protein production method using *Escherichia coli* (Non-Patent Literature 2).

CITATION LIST

- Non-Patent Literature

Non-Patent Literature 1: Tadashi Baba, "Kozoseibutsu" (structural biology), Vol. 5, No. 1 (1998), pp. 35-39
Non-Patent Literature 2: Nobuyuki Esaki, et al. "Seikagaku kiso no kiso" (introduction to basic biochemistry), Kagaku-Dojin Publishing Co., Inc. March, 2002, pp. 288-289

SUMMARY OF INVENTION

- Technical Problem

The present invention aims to provide a high yield method of producing useful substances using yeast.

- Solution to Problem

As a result of extensive studies to solve the above problems, the present inventors arrived at the present invention. Specifically, the present invention provides a method of producing a useful substance, including secreting and producing a useful substance in broth by yeast contained in the broth, the yeast being a *Saccharomyces* strain, the broth containing a compound (A) represented by a formula (1), the compound (A) having an HLB value of 0.1 to 16 and a number average molecular weight of 200 to 30000, the weight percentage of the compound (A) based on the weight of the broth being 0.0001 to 10 wt%:

$$\text{HO-}(A^1O)m^1\text{-}(A^2O)m^2\text{-}(A^3O)m^3\text{-H} \qquad (1)$$

wherein $A^1O$, $A^2O$, and $A^3O$ each independently represent a C2-C4 oxyalkylene group; $A^1O$ and $A^2O$ have different structural formulas, $A^2O$ and $A^3O$ have different structural formulas;
and $m^1$, $m^2$, and $m^3$ each respectively represent the average number of moles of $A^1O$, $A^2O$, and $A^3O$ added, and are each independently a number in the range of 1 to 600.

- Advantageous Effects of Invention

The method of producing a useful substance of the present invention can produce a large amount of useful substances using yeast.

BRIEF DESCRIPTION OF DRAWINGS

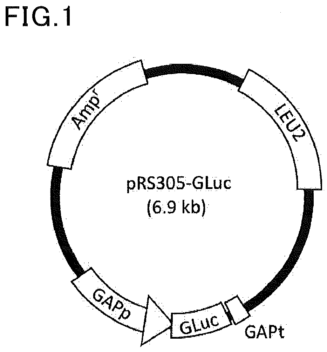

Fig. 1 shows a vector that is used in Production Example 12.

DESCRIPTION OF EMBODIMENTS

The method of producing a useful substance of the present invention includes secreting and producing a useful substance in broth by yeast contained in the broth, the broth containing a compound (A) represented by the following formula (1), the compound (A) represented by the following formula (1) having an HLB value of 0.1 to 16 and a number average molecular weight of 200 to 30000, the broth containing the compound (A) represented by the following formula (1) in an amount of 0.0001 to 10 wt% based on the weight of the broth:

$$HO\text{-}(A^1O)m^1\text{-}(A^2O)m^2\text{-}(A^3O)m^3\text{-}H \qquad (1)$$

wherein $A^1O$, $A^2O$, and $A^3O$ each independently represent a C2-C4 oxyalkylene group; $A^1O$ and $A^2O$ have different structural formulas, $A^2O$ and $A^3O$ have different structural formulas;
and $m^1$, $m^2$, and $m^3$ each respectively represent the average number of moles of $A^1O$, $A^2O$, and $A^3O$ added, and are each independently a number in the range of 1 to 600.

Any culture medium can be used in the production method of the present invention as long as it is a cell culture medium usually used in the relevant technical field. Either a natural medium or synthetic medium containing a carbon source, a nitrogen source, and other essential nutrients may be used.

Examples of the carbon source include carbohydrates such as glucose, fructose, sucrose, and starch; organic acids such as acetic acid and propionic acid; and alcohols such as ethanol and propanol.

Examples of the nitrogen source include ammonium salts of inorganic acids or organic acids (e.g., ammonium chloride, ammonium sulfate, ammonium acetate, and ammonium phosphate), ammonia, peptone, meat extract, and corn steep liquor.

Examples of the other essential nutrients include inorganic salts. Examples of the inorganic salts include monopotassium phosphate, dipotassium phosphate, magnesium phosphate, magnesium sulfate, sodium chloride, ferrous sulfate, manganese sulfate, copper sulfate, and calcium carbonate.

The production method uses a *Saccharomyces* strain because it can be genetically modified and is easily applicable for industrial use.

Preferred examples of the *Saccharomyces* strain include *Saccharomyces cerevisiae, Saccharomyces bayanus, Saccharomyces kudriavzevii, Saccharomyces mikatae, Saccharomyces paradoxus,* and *Saccharomyces pastorianus.*

The yeast for use in the production method of the present invention may be yeast that has been genetically modified according to the type of a target useful substance.

Examples of the genetic modification method include a method described below in which bacteria (e.g., *Escherichia coli* and yeast) are used to construct plasmids to be introduced into yeast, and the constructed plasmids are introduced into the yeast for transformation.

(1) Construction of plasmid to be introduced into yeast

A plasmid to be introduced into yeast can be constructed by the following method, for example.

Messenger RNA (mRNA) is isolated from cells (e.g., animal cells and plant cells) expressing the target protein, single-stranded cDNA is produced from the mRNA, and double-stranded DNA is then synthesized. Subsequently, the synthesized double-stranded DNA is introduced into phage DNA or plasmids to be introduced into bacteria. The resulting recombinant phage or plasmids are introduced into host bacteria for transformation, and a cDNA library is produced.

Next, the cDNA library prepared from the transformed bacteria is screened for plasmids containing the target DNA. Screening can be performed by, for example, hybridization of plasmids with the target protein gene or DNA probes partially encoding a complementary sequence.

After the screening, a cloned DNA or a part thereof is cut out from a plasmid containing the target cloned DNA in the bacteria, and the cloned DNA or a part thereof is located downstream of a promoter, whereby a plasmid containing the target gene to be introduced into yeast can be constructed.

When introducing the cloned DNA or a part thereof into a plasmid to be introduced into yeast, DNA encoding a signal sequence to be translocated across the inner membrane (i.e., a signal sequence to express the target substance in the periplasmic space) can also be located downstream of the promoter.

Such a plasmid to be introduced into yeast can be constructed, for example, by the method described in Baio jikken irasutoreiteddo 7 (biology experiments illustrated 7) (Takayuki Mizuno, published on October 30, 2003), pp. 47-69.

(2) Transformation of yeast

Yeast can be transformed using the plasmid to be introduced into yeast which is constructed in (1) above, by a method in which yeast is treated with a lithium salt to allow DNA to be naturally easily introduced thereinto before the plasmid is introduced or a method in which DNA is introduced into cells by electroporation (Becker and Guarente, Methods Enzymol., Vol. 194, pp. 182-187 (1991)).

The broth used in the production method of the present invention contains the compound (A) represented by the formula (1). The compound (A) may be used alone or in combination of two or more thereof.

In the formula (1), $A^1O$, $A^2O$, and $A^3O$ each independently represent a C2-C4 oxyalkylene group.

$A^1O$ and $A^2O$ have different structural formulas, and $A^2O$ and $A^3O$ have different structural formulas.

Examples of the C2-C4 oxyalkylene group include an oxyethylene group (hereinafter abbreviated as EO), a 1,2- or 1,3-oxypropylene group (hereinafter abbreviated as PO), and a 1,2-, 1,3-, 1,4- or 2,3-oxybutylene group (hereinafter abbreviated as BO).

In terms of useful substance yield, $A^1O$, $A^2O$, and $A^3O$ are preferably each independently EO or PO. A more preferred combination is one in which $A^1O$ and $A^3O$ are EO and $A^2O$ is PO.

In terms of useful substance yield, PO in the above combination is preferably a 1,2-oxypropylene group.

In the formula (1), $m^1$, $m^2$, and $m^3$ each respectively represent the average number of moles of $A^1O$, $A^2O$, and $A^3O$ added, and are each a number in the range of 1 to 600.

In terms of useful substance yield, preferably, $m^1$ and $m^3$ are each a number in the range of 1 to 200 and $m^2$ is a number in the range of 10 to 70.

The compound (A) represented by the formula (1) for use in the method of the present invention has an HLB value of 0.1 to 16, preferably 9 to 16. An HLB value outside the range of 0.1 to 16 results in low useful substance yield.

The HLB value of the compound (A) represented by the formula (1) is an indicator that indicates the hydrophiliclipophilic balance. The HLB value can be calculated by the following formula from the ratio of the molecular weight of the hydrophilic moiety of the compound represented by the formula (1) to the molecular weight of the compound (A) represented by the formula (1) by Griffin's method described in Kaimen kasseizai nyumon (Introduction to Surfactants) (Takehiko Fujimoto, published by Sanyo Chemical Industries, Ltd. 2007), p. 142.

$$\text{HLB value} = 20 \times \text{molecular weight of hydrophilic moiety of compound (A)/molecular weight of compound (A)}$$

In the method of the present invention, the number average molecular weight of the compound (A) represented by the formula (1) is 200 to 30000, and it is preferably 1000 to 20000 in terms of useful substance yield.

In the method of the present invention, the number average molecular weight of the compound (A) represented by the formula (1) can be calculated by the following method.

The number average molecular weight can be measured from the content soluble in tetrahydrofuran (hereinafter abbreviated as THF) by gel permeation chromatography (GPC) under the following conditions.

Measurement device: "HLC-8120" (available from Tosoh Corporation)

Column: "TSKgel GMHXL" (two columns) and "TSKgel Multipore HXL-M" (one column) (all available from Tosoh Corporation)

Sample solution: 0.25 mass% solution in THF

Amount of sample solution into columns: 100 μl

Flow rate: 1 ml/min

Measurement temperature: 40°C

Detector: refractive index detector

Standard substance: Standard polystyrene available from Tosoh Corporation (TSK standard polystyrene), 12 samples (number average molecular weight: 500, 1050, 2800, 5970, 9100, 18100, 37900, 96400, 190000, 355000, 1090000, and 2890000)

In the production method of the present invention, the timing to add the compound (A) represented by the formula (1) is not limited as long as it is contained in the broth at some point before or during yeast cultivation.

For example, the compound (A) represented by the formula (1) may be added to the broth prior to starting or during culturing.

In terms of production efficiency and the like of the useful substance, preferably, the broth at the time of starting culturing does not contain the compound (A) represented by the formula (1). In this case, the compound (A) represented by the formula (1) is preferably added during the logarithmic growth phase of the yeast.

In terms of solubility, the compound (A) represented by the formula (1) is preferably added in the form of aqueous solution to the broth.

In the method of the present invention, the total amount (wt%) of the compound (A) represented by the formula (1) may be determined as appropriate according to the types of microorganisms to be used and target useful substances to be produced and the type of extraction method. Yet, the amount of the compound (A) is 0.0001 to 10 wt% based on the weight of the broth, and it is preferably 0.005 to 10 wt% in order to inhibit denaturation of the protein produced.

The weight of the broth is based on its weight at the time of starting culturing.

Examples of the useful substance produced by the method of producing a useful substance of the present invention include proteins (e.g., enzymes, hormone proteins, antibodies, and peptides), polysaccharides, oligosaccharides, and nucleic acids.

Examples of proteins include enzymes such as oxidoreductases (e.g., cholesterol oxidase, glucose oxidase, ascorbic acid oxidase, and peroxidase), hydrolases (e.g., lysozyme, protease, serine protease, amylase, lipase, cellulase, and glucoamylase), isomerases (e.g., glucose isomerase), transferases (e.g., acyltransferase and sulfotransferase), synthetases (fatty acid synthase, phosphate synthase, and citrate synthase), and lyases (e.g., pectin lyase); hormone proteins such as osteogenic factor (BMP), interferon $\alpha$, interferon $\beta$, interleukins 1 to 12, growth hormone, erythropoietin, insulin, granulocyte-colony stimulating factor (G-CSF), tissue plasminogen activator (TPA), natriuretic peptide, factor VIII, somatomedin, glucagon, growth hormone-releasing factor, serum albumin, and calcitonin; antibodies; antigen proteins (e.g., hepatitis B surface antigen); functional proteins such as pronectin (registered trademark), antifreeze peptide, and antimicrobial peptide; fluorescent protein (e.g., GFP); luminescent proteins (e.g., luciferase); and peptides (the amino acid composition is not limited; e.g., oligopeptide, dipeptide, and tripeptide).

Examples of polysaccharides include hyaluronic acid, chondroitin, xanthan, and cellulose.

Examples of oligosaccharides include sucrose, lactose, trehalose, maltose, raffinose, panose, cyclodextrin, galactooligosaccharides, and fructooligosaccharides.

Examples of nucleic acids include inosine monophosphate, adenosine monophosphate, and guanosine monophosphate.

In terms of production efficiency and the like of the useful substance, proteins are preferred.

The method of producing a useful substance of the present invention preferably includes a culturing step and a purification step.

In the culturing step, preferably, broth to be used in the production of the useful substance is sterilized in an autoclave (preferably, heating at 120°C for 20 minutes), and precultured yeast is cultured in this broth.

The temperature for yeast cultivation is preferably 15°C to 32°C, more preferably 25°C to 30°C.

The pH of the medium is preferably 4 to 7.

Preferably, the culturing step is performed as follows, for example: an aqueous solution of the compound (A) represented by the formula (1) is added to the broth 6 to 800 hours after the start of culturing while the broth temperature is maintained, and the culturing is continued for 20 to 1000 hours.

The broth during culturing is preferably stirred using a known stirrer (e.g., a stirring blade or a magnetic stirrer). A known culture device can be used in the culturing step. Examples include test tubes, deep well plates (e.g., products of BM Equipment Co., Ltd.), and microbial culture devices (e.g., products of ABLE Corporation).

When recombinant yeast is used as the precultured yeast to be used in the culturing step, the recombinant yeast is produced by transforming yeast with an expression vector and the produced recombinant yeast is precultured. Preferably, the preculturing is performed in an agar medium at 15°C to 32°C for 3 to 72 hours.

The purification step is a step of isolating the useful substance (e.g., protein) secreted in the broth. The useful substance can be separated from microorganisms and microbial residues by a known separation method such as precipitation (e.g., centrifugal separation, hollow fiber separation, filtration, or precipitation by solvent) or column chromatography treatment (ion exchange column, gel filtration column, hydrophobic column, affinity column, or ultrafiltration column.

Examples of precipitation by solvent include ethanol precipitation, ammonium sulfate precipitation, and polyethylene glycol precipitation.

When the purification step involves column chromatography treatment, examples of filler to be used in the column chromatography include silica, dextran, agarose, cellulose, acrylamide, and vinyl polymer. Commercial products such as Sephadex series, Sephacryl series, Sepharose series (all available from Pharmacia), and BioGel series (available from Bio-Rad) are available.

The yeast isolated in the purification step can be further cultured in fresh broth. The useful substance can be produced continuously by repeating purifying the broth or the like in the purification step and culturing.

EXAMPLES

The present invention is described in further detail with reference to the following examples and comparative examples, but the present invention is not limited thereto. Hereinafter, "part(s)" means" part(s) by weight", unless otherwise specified.

Compounds (A-1) to (A-11) were produced according to the following production methods, as the compound (A) represented by the formula (1) to be used in the examples and the comparative examples.

Table 1 shows the number average molecular weight and the HLB value of each of the compounds (A-1) to (A-11) produced.

<Production Example 1>

To a stainless steel pressure reactor were added 1,2-propanediol (19 parts) and sodium hydroxide (0.05 parts). After nitrogen purging, 1,2-propylene oxide (420 parts) was gradually added dropwise over 4 hours at 110°C to 130°C such that the inner pressure of the autoclave was kept below 0.3 MPa. The reaction was continued for additional 10 hours at the same temperature.

Subsequently, ethylene oxide (66 parts) was gradually added dropwise over 4 hours at the same temperature such that the inner temperature of the autoclave was kept below 0.3 MPa, and then the reaction was continued for additional 10 hours at the same temperature. Thus, a compound (A-1) was obtained.

<Production Example 2>

A compound (A-2) was obtained by the same procedure as in Production Example 1, except that the amount of 1,2-propanediol was changed from 19 parts to 15.8 parts, the amount of 1,2-propylene oxide was changed from 420 parts to 350 parts, and the amount of ethylene oxide was changed from 66 parts to 138 parts.

<Production Example 3>

A compound (A-3) was obtained by the same procedure as in Production Example 1, except that the amount of 1,2-propanediol was changed from 19 parts to 12.2 parts, the amount of 1,2-propylene oxide was changed from 420 parts to 270 parts, and the amount of ethylene oxide was changed from 66 parts to 220 parts.

<Production Example 4>

A compound (A-4) was obtained by the same procedure as in Production Example 1, except that the amount of 1,2-propanediol was changed from 19 parts to 4.8 parts, the amount of 1,2-propylene oxide was changed from 420 parts to 106 parts, and the amount of ethylene oxide was changed from 66 parts to 394 parts.

<Production Example 5>

To a stainless steel pressure reactor were added SANNIX PP-2000 (Sanyo Chemical Industries, Ltd.; polyoxypropylene glycol whose constituent unit is a 1,2-oxypropylene group (number average molecular weight: 2000)) (450 parts) and sodium hydroxide (0.05 parts). After nitrogen purging, ethylene oxide (50 parts) was gradually added dropwise over 4 hours at 110°C to 130°C such that the inner pressure of the autoclave was kept below 0.3 MPa, and the reaction was then continued for additional 10 hours at the same temperature. Thus, a compound (A-5) was obtained.

<Production Example 6>

A compound (A-6) was obtained by the same procedure as in Production Example 5, except that the amount of SANNIX PP-2000 was changed from 450 parts to 323 parts, and the amount of ethylene oxide was changed from 50 parts to 178 parts.

<Production Example 7>

A compound (A-7) was obtained by the same procedure as in Production Example 5, except that the amount of SANNIX PP-2000 was changed from 450 parts to 106 parts, and the amount of ethylene oxide was changed from 50 parts to 392 parts.

<Production Example 8>

A compound (A-8) was obtained by the same procedure as in Production Example 5, except that SANNIX PP-3000 (Sanyo Chemical Industries, Ltd.; polyoxypropylene glycol whose constituent unit is a 1,2-oxypropylene group (number average molecular weight: 3200)) (101 parts) was used instead of SANNIX PP-2000 (450 parts) and the amount of ethylene oxide was changed from 50 parts to 402 parts.

<Production Example 9>

A compound (A-9) was obtained by the same procedure as in Production Example 1, except that the amount of 1,2-propylene oxide was changed from 420 parts to 290 parts, and the amount of ethylene oxide was changed from 66 parts to 250 parts.

<Production Example 10>

To a stainless steel pressure reactor were added ethylene glycol (10 parts) and sodium hydroxide (0.05 parts). After nitrogen purging, ethylene oxide (35 parts) was gradually added dropwise over 4 hours at 110°C to 130°C such that the inner pressure of the autoclave was kept below 0.3 MPa. The reaction was continued for additional 10 hours at the same temperature.
Subsequently, 1,2-propylene oxide (402 parts) was gradually added dropwise over 4 hours at the same temperature such that the inner temperature of the autoclave was kept below 0.3 MPa. The reaction was continued for additional 10 hours at the same temperature. Thus, a compound (A-10) was obtained.

<Production Example 11>

A compound (A-11) was obtained by the same procedure as in Production Example 10, except that the amount of ethylene oxide was changed from 35 parts to 92 parts.

[Table 1]

| | Compound (A) represented by formula (1) | Number average molecular weight | HLB | $A^1O$ | $A^2O$ | $A^3O$ | $m^1$ | $m^2$ | $m^3$ |
|---|---|---|---|---|---|---|---|---|---|
| | | | | EO or PO | | | Number of moles added | | |
| Production Example 1 | A-1 | 2000 | 2.5 | EO | PO | EO | 3.0 | 30 | 3.0 |
| Production Example 2 | A-2 | 2400 | 5.5 | EO | PO | EO | 7.5 | 30 | 7.5 |
| Production Example 3 | A-3 | 3100 | 8.7 | EO | PO | EO | 16 | 30 | 16 |
| Production Example 4 | A-4 | 8000 | 15.6 | EO | PO | EO | 71 | 30 | 71 |
| Production Example 5 | A-5 | 2200 | 1.8 | EO | PO | EO | 2.5 | 34 | 2.5 |
| Production Example 6 | A-6 | 3100 | 7.2 | EO | PO | EO | 13 | 34 | 13 |
| Production Example 7 | A-7 | 9400 | 15.8 | EO | PO | EO | 84 | 34 | 84 |
| Production Example 8 | A-8 | 16000 | 15.9 | EO | PO | EO | 145 | 55 | 145 |
| Production Example 9 | A-9 | 2200 | 8.9 | EO | PO | EO | 11 | 21 | 11 |
| Production Example 10 | A-10 | 2800 | 2.0 | PO | EO | PO | 22 | 6.0 | 22 |

(continued)

| | Compound (A) represented by formula (1) | Number average molecular weight | HLB | A$^1$O | A$^2$O | A$^3$O | m$^1$ | m$^2$ | m$^3$ |
|---|---|---|---|---|---|---|---|---|---|
| | | | | EO or PO | | | Number of moles added | | |
| Production Example 11 | A-11 | 3100 | 4.0 | PO | EO | PO | 22 | 14 | 22 |

The number average molecular weight of each of the compounds (A) represented by the formula (1) used in the examples and the comparative examples was measured by the following method.

The number average molecular weight was measured from the content soluble in tetrahydrofuran (hereinafter abbreviated as THF) by gel permeation chromatography (GPC) under the following conditions.

Measurement device: "HLC-8120" (available from Tosoh Corporation)

Column: "TSKgel GMHXL" (two columns) and "TSKgel Multipore HXL-M" (one column) (all available from Tosoh Corporation)

Sample solution: 0.25 mass% solution in THF

Amount of sample solution into columns: 100 μl

Flow rate: 1 ml/min

Measurement temperature: 40°C

Detector: refractive index detector

Standard substance: Standard polystyrene available from Tosoh Corporation (TSK standard polystyrene), 12 samples (number average molecular weight: 500, 1050, 2800, 5970, 9100, 18100, 37900, 96400, 190000, 355000, 1090000, and 2890000)

<Production Example 12: Production of yeast>

The amino acid sequence of luciferase (GLuc) derived from *Gaussia princeps* is disclosed in AAG54095 on UniProt.

Based on the amino acid sequence, a gene sequence to match the codon usage in budding yeast was designed, and a gene (hereinafter, SEQ ID No: 1) encoding a luciferase protein derived from *Gaussia princeps* was synthesized.

The thus-obtained synthetic gene was digested with commercially available EcoRI and SalI, and a vector YEp352GAP-II was digested with commercially available EcoRI and SalI. Subsequently, these samples were subjected to agarose gel electrophoresis, and bands corresponding to fragments of these samples were cut out from the gel. DNA fragments were extracted from the cut-out gel and purified, using Wizard SV DNA and PCR Clean-Up System (Promega Corporation). The DNA concentration was measured with NanoDrop Lite (Thermo Fisher Scientific). Subsequently, the fragments (50 ng, each) were mixed together, and H$_2$O was added to the mixture to obtain an amount of 7.5 μL. Then, 3.75 μL of Ligation High Ver. 2 (TOYOBO) was added for reaction at 16°C for 30 minutes. Using 2 μL of the reaction product, ECOS™ Competent Escherichia coli. DH5α (Nippon Gene Co., Ltd.) was transformed. The transformed *Escherichia coli* was cultured in LB-agar medium containing 50 μg/mL of ampicillin at 37°C for 16 hours. Thus, a transformant was obtained.

The resulting colonies were cultured in LB liquid medium containing 50 μg/mL of ampicillin at 37°C for 16 hours. A vector YEp352GAP-II-GLuc was purified by Wizard Plus SV Minipreps DNA Purification System (Promega Corporation) from the bacteria. The sequence was analyzed to make sure that there was no error in the base sequence. The vector YEp352GAP-II-GLuc was thus obtained.

A region containing GAPDH promoter and terminator was collected using BamHI from the thus-obtained YEp352GAP-II-GLuc vector in the same manner as described above, and was similarly inserted into the BamHI site of yeast-integrated plasmid pRS305 for transformation. The resulting colonies were cultured, and the plasmid was then collected. Thus, a vector (pRS305-GLuc) shown in Fig. 1 was produced. This vector (pRS305-GLuc) was digested with EcoRV and linearized. Subsequently, a budding yeast (*Saccharomyces cerevisiae*) W303-1B strain was transformed according to a usual method.

The bacterial solution after transformation was applied to SD-Leu agar medium (2.67% Minimal SD Base Medium, 0.069% DO Supplement-Leu (TaKaRa Bio), 2% Agar), and cultured at 30°C for 2 days. Thus, colonies of the transformant were obtained. The colonies were scraped from the plate, and were suspended in a PCR reaction solution according to a simplified PCR method to make sure that the colonies were introduced into chromosomes. The thus-obtained yeast (*Saccharomyces* strain) was regarded as GLuc-expressing budding yeast.

<Examples 1 to 10>

The GLuc-expressing budding yeast produced in Production Example 12 was inoculated using a platinum loop into 5 ml of casamino acid medium (0.5 wt% casamino acid (technical Difco™, Becton, Dickinson and Company (hereinafter, BD)); 0.67 wt% yeast nitrogen base (Yeast Extract Difco™, BD); and 2 wt% glucose (D(+)-glucose, Wako Pure Chemical Industries, Ltd.)), and was shake-cultured at 200 rpm at 30°C for 6 hours. Then, 15 μl of the thus-obtained broth was resuspended in 2 ml of YPAD medium (2 wt% peptone (Bacto Peptone Difco™, BD); 1 wt% yeast extract (Yeast Extract Difco™, BD) ; 2 wt% glucose (D (+) - glucose, Wako Pure Chemical Industries, Ltd.)); and adenine (Adenine Sulfate, Wako Pure Chemical Industries, Ltd. (40 mg/l).

The broth obtained by resuspension was continuously shake-cultured at 1100 rpm at 30°C for 2 hours. Then, the compound (A) described in Table 2 was added in an amount of 0.3 wt% based on the weight of the broth.

Subsequently, the broth was continuously shake-cultured at 1100 rpm at 30°C for additional 24 hours.

Subsequently, the broth was sampled, and the broth containing yeast was subjected to centrifugation using a centrifuge to separate the bacteria and the supernatant from the broth. Then, the supernatant was collected.

<Comparative Example 1>

The culture supernatant was obtained in the same manner as in Example 1, except that the compound (A) was not added.

<Examples 11 to 14>

In each of these examples, supernatant was obtained in the same manner as in Example 8, except that the compound (A-8) was added in an amount (wt%) described in Table 3, based on the weight of the broth.

<Evaluation of optical density of broth>

The supernatant was placed in a 1-ml quartz cell (light path of 10 mm), and the optical density was measured using a spectrophotometer (UV-1700, Shimadzu Corporation).

The supernatant was diluted in a saline solution to obtain an appropriate absorbance.

Additionally, 2 ml of YPAD medium was used as a blank for the supernatant obtained in each of Examples 1 to 14. The optical density of the broth was calculated by the following mathematical formula (1).

```
Optical density of broth = [(Measured value of optical
density of diluted broth) - (Measured value of optical
density of blank)] × dilution factor of broth
[Mathematical formula (1)]
```

For each of Examples 1 to 14 and Comparative Example 1, the "optical density of broth" was divided by the optical density of the broth in Comparative Example 1, and the obtained value was regarded as the broth optical density ratio. Table 2 and Table 3 show the evaluation results.

A higher broth optical density ratio indicates a higher yeast concentration.

Additionally, a higher protein activity value per yeast concentration indicates a higher production efficiency of proteins whose activities are maintained.

<Luciferase activity evaluation>

The luciferase activity can be evaluated by measuring the luciferase activity of a mixture of the cultivation supernatant (20 μl) of the luciferase-expressing yeast with a luciferin solution (60 μl) using a luminometer under the following conditions.

Luminometer: "AB-2350 PHELIOS" available from Atto Corporation

Luciferin solution: a product available from New England Biolabs Japan Inc. or a product available from Atto Corporation

Sample solution: supernatant obtained by culturing luciferase-expressing yeast for a predetermined period of time according to the production example

Measurement temperature: 25°C

Detector: emission detector

Detection wavelength: 460 nm

For each of Examples 1 to 14 and Comparative Example 1, the measured emission intensity was divided by the emission intensity in Comparative Example 1, and the obtained value was regarded as the luciferase activity value ratio. Table 2 and Table 3 show the evaluation results.

A higher luciferase activity value ratio indicates a higher protein activity value per unit volume of broth supernatant.

The luciferase activity value ratio per yeast concentration can be determined by dividing the luciferase activity ratio of an example or comparative example by the broth optical density ratio of the corresponding example or comparative example.

A higher luciferase activity value ratio per yeast concentration indicates a higher production efficiency of proteins whose luciferase activities are maintained.

<Evaluation of luciferase expression level>

The luciferase expression level was evaluated by Western blotting (including electrophoresis, transfer, and luminescence) described in detail below.

(1) Electrophoresis

Electrophoresis was performed using a mixture of the cultivation supernatant (10 µl) of the recombinant yeast with a sample buffer solution (10 µl) under the following conditions.
Sample solution: supernatant obtained in each example or comparative example
Polyacrylamide gel: E-R15L e-PAGEL (Atto Corporation)
Electrophoresis tank: WSE-1100P PageRun-R (Atto Corporation)
Electrophoresis time: 90 min
Sample buffer solution: a mixture of 4x Laemmli sample buffer (Bio-Rad Laboratories, Inc.) (900 µl) and 2-mercaptoethanol (100 µl)

(2) Transfer

Transfer can be performed using a transfer membrane under the following conditions, after performing the electrophoresis under the above conditions.
Transfer membrane: Amersham Hybond P0.45 PVDF (GE Healthcare)
Transfer solution: a solution obtained by 10-fold dilution of AE-1465 EzFastBlot (Atto Corporation) with distilled water
Voltage/current: 12 V/400 mA
Transfer time: 30 min

(3) Luminescence

After the transfer under the above conditions, the transfer membrane was blocked, and antigen-antibody reaction was then performed with primary and secondary antibodies. In this test, the primary antibody was an anti-Gluc antibody (BioLabs), and the secondary antibody was a horseradish peroxidase (HRP)-labeled secondary antibody. The luminescence was detected using ImageQuant LAS 4000 under the following conditions.
Detector: ImageQuant LAS 4000 (a product available from Fujifilm Corporation)
Blocking time: 45 min
Primary antibody reaction time: 120 min
Secondary antibody reaction time: 60 min

(4) Expression level analysis method

After a band corresponding to the luciferase was detected on the transfer membrane after performing the steps (1) to (3), the intensity of the band was determined quantitatively using ImageJ (National Institutes of Health, USA).

For each of Examples 1 to 14 and Comparative Example 1, the intensity of the band was divided by the intensity of the band in Comparative Example 1, and the obtained value was regarded as the luciferase expression level ratio. Table 2 and Table 3 show the evaluation results.

A higher luciferase expression level ratio indicates a higher luciferase expression level per unit volume of broth supernatant.

The luciferase expression level ratio per yeast concentration can be determined by dividing the luciferase expression level ratio of an example or comparative example by the broth optical density ratio of the corresponding example or comparative example.

A higher luciferase expression level ratio per yeast concentration indicates a higher production efficiency of proteins whose luciferase activities are maintained.

[Table 2]

| | | Example | | | | | | | | | | Comparative Example |
| --- | --- | --- | --- | --- | --- | --- | --- | --- | --- | --- | --- | --- |
| | Compound (A) | 1 | 2 | 3 | 4 | 5 | 6 | 7 | 8 | 9 | 10 | 1 |
| Amount of compound (A) (wt%) | A-1 | 0.3 | - | - | - | - | - | - | - | - | - | - |
| | A-2 | - | 0.3 | - | - | - | - | - | - | - | - | - |
| | A-3 | - | - | 0.3 | - | - | - | - | - | - | - | - |
| | A-4 | - | - | - | 0.3 | - | - | - | - | - | - | - |
| | A-5 | - | - | - | - | 0.3 | - | - | - | - | - | - |
| | A-6 | - | - | - | - | - | 0.3 | - | - | - | - | - |
| | A-7 | - | - | - | - | - | - | 0.3 | - | - | - | - |
| | A-8 | - | - | - | - | - | - | - | 0.3 | - | - | - |
| | A-10 | - | - | - | - | - | - | - | - | 0.3 | - | - |
| | A-11 | - | - | - | - | - | - | - | - | - | 0.3 | - |
| Evaluation results | Luciferase expression level ratio | 1.6 | 1.5 | 1.1 | 1.4 | 1.3 | 1.3 | 1.6 | 1.6 | 1.3 | 1.4 | 1.0 |
| | Luciferase activity value ($\times 10^6$ RLU) | 6.1 | 5.5 | 5.3 | 4.7 | 5.2 | 5.1 | 5.7 | 8.1 | 4.4 | 5.7 | 4.0 |
| | Luciferase activity value ratio | 1.5 | 1.4 | 1.3 | 1.2 | 1.3 | 1.3 | 1.4 | 2.0 | 1.1 | 1.4 | 1.0 |
| | Broth optical density (absorbance) | 0.84 | 0.81 | 0.77 | 0.71 | 0.79 | 0.76 | 0.68 | 0.68 | 0.77 | 0.75 | 0.79 |
| | Broth optical density ratio | 1.06 | 1.02 | 0.98 | 0.90 | 1.01 | 0.97 | 0.86 | 0.86 | 0.98 | 0.95 | 1.00 |
| | Luciferase expression level per yeast concentration (expression level ratio/turbidity ratio) | 1.5 | 1.5 | 1.1 | 1.5 | 1.3 | 1.3 | 1.8 | 1.8 | 1.3 | 1.5 | 1.0 |
| | Luciferase activity value per yeast concentration (activity value ratio/turbidity ratio) | 1.4 | 1.3 | 1.4 | 1.3 | 1.3 | 1.3 | 1.7 | 2.4 | 1.1 | 1.5 | 1.0 |

[Table 3]

| | Compound (A) | Example | | | | Comparative Example |
|---|---|---|---|---|---|---|
| | | 11 | 12 | 13 | 14 | 1 |
| Amount of compound (A) (wt%) | Compound (A-8) (wt%) | 0.1 | 0.2 | 0.3 | 0.45 | - |
| Evaluation results | Luciferase expression level ratio | 1.2 | 1.2 | 1.6 | 1.3 | 1.0 |
| | Luciferase activity value (x10$^6$ RLU) | 8.4 | 8.4 | 8.1 | 11.3 | 4.0 |
| | Luciferase activity value ratio | 2.1 | 2.1 | 2.0 | 2.8 | 1.0 |
| | Broth optical density (absorbance) | 0.77 | 0.81 | 0.68 | 0.82 | 0.79 |
| | Broth optical density ratio | 0.98 | 1.02 | 0.86 | 1.04 | 1.00 |
| | Luciferase expression level per yeast concentration (expression level ratio/ optical density ratio) | 1.2 | 1.2 | 1.8 | 1.3 | 1.0 |
| | Luciferase activity value per yeast concentration (activity value ratio/optical density ratio) | 2.1 | 2.1 | 2.4 | 2.7 | 1.0 |

Clearly, the products of Examples 1 to 14 each containing the compound (A) represented by the formula (1) have a higher yield of the enzyme (luciferase) which is a protein provided as the useful substance than the product of Comparative Example 1 not containing the compound (A).

INDUSTRIAL APPLICABILITY

The method of producing a useful substance of the present invention is useful for efficiently producing biopharmaceuticals.

REFERENCE SIGNS LIST

    Amp$^r$: ampicillin resistance gene
LEU2: leucine synthetase
GAPp: GAP promoter
GAPt: GAP terminator
GLuc: luciferase derived from *Gaussia princeps*

SEQUENCE LISTING FREE TEXT

# EP 3 591 061 A1

## Claims

1.  A method of producing a useful substance, comprising:
    secreting and producing a useful substance in broth by yeast contained in the broth, the yeast being a *Saccharomyces* strain, the broth containing a compound (A) represented by a formula (1), the compound (A) having an HLB value of 0.1 to 16 and a number average molecular weight of 200 to 30000, the weight percentage of the compound (A) based on the weight of the broth being 0.0001 to 10 wt%:

    $$HO\text{-}(A^1O)m^1\text{-}(A^2O)m^2\text{-}(A^3O)m^3\text{-}H \qquad (1)$$

    wherein $A^1O$, $A^2O$, and $A^3O$ each independently represent a C2-C4 oxyalkylene group; $A^1O$ and $A^2O$ have

different structural formulas, $A^2O$ and $A^3O$ have different structural formulas;
and $m^1$, $m^2$, and $m^3$ each respectively represent the average number of moles of $A^1O$, $A^2O$, and $A^3O$ added, and are each independently a number in the range of 1 to 600.

2. The method of producing a useful substance according to claim 1,
   wherein the useful substance is a protein.

3. The method of producing a useful substance according to claim 1 or 2,
   wherein $A^1O$, $A^2O$, and $A^3O$ in the compound (A) represented by the formula (1) each independently represent an oxyethylene group or a 1,2-oxypropylene group.

4. The method of producing a useful substance according to any one of claims 1 to 3,
   wherein $A^1O$ and $A^3O$ are oxyethylene groups and $A^2O$ is a 1,2-oxypropylene group in the compound (A) represented by the formula (1).

5. The method of producing a useful substance according to any one of claims 1 to 4,
   wherein the compound (A) represented by the formula (1) has a number average molecular weight of 1000 to 20000, $A^2O$ is a 1,2-oxypropylene group, and $m^2$ is a number in the range of 10 to 70.

FIG.1

pRS305-GLuc
(6.9 kb)

# EP 3 591 061 A1

## INTERNATIONAL SEARCH REPORT

| | International application No. |
|---|---|
| | PCT/JP2018/007420 |

### A. CLASSIFICATION OF SUBJECT MATTER
Int.Cl. C12P1/02(2006.01)i, C12P21/00(2006.01)i, C12P21/02(2006.01)i

According to International Patent Classification (IPC) or to both national classification and IPC

### B. FIELDS SEARCHED

Minimum documentation searched (classification system followed by classification symbols)
Int.Cl. C12P1/02, C12P21/00, C12P21/02

Documentation searched other than minimum documentation to the extent that such documents are included in the fields searched
| | |
|---|---|
| Published examined utility model applications of Japan | 1922–1996 |
| Published unexamined utility model applications of Japan | 1971–2018 |
| Registered utility model specifications of Japan | 1996–2018 |
| Published registered utility model applications of Japan | 1994–2018 |

Electronic data base consulted during the international search (name of data base and, where practicable, search terms used)
JSTPlus/JMEDPlus/JST7580 (JDreamIII), CAplus/MEDLINE/BIOSIS/WPIDS (STN)

### C. DOCUMENTS CONSIDERED TO BE RELEVANT

| Category* | Citation of document, with indication, where appropriate, of the relevant passages | Relevant to claim No. |
|---|---|---|
| X | JP 60-196185 A (GENERAL INCORPORATED FOUNDATION, THE CHEMO-SERO-THERAPEUTIC RESEARCH INSTITUTE) 04 October 1985, claims, examples & EP 157275 A1, claims, examples & CA 1277264 A & KR 10-1992-0007680 B | 1–5 |
| Y | | 1–5 |
| X | JP 2002-291494 A (TOYOTA MOTOR CORP.) 08 October 2002, example 1, fig. 3 & US 2003/0096385 A1, example 1, fig. 3 & EP 1219704 A2 | 1, 3–5 |
| Y | | 1–5 |

☒ Further documents are listed in the continuation of Box C.  ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 24 May 2018 (24.05.2018) | 05 June 2018 (05.06.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**INTERNATIONAL SEARCH REPORT**

| International application No. |
| --- |
| PCT/JP2018/007420 |

C (Continuation).  DOCUMENTS CONSIDERED TO BE RELEVANT

| Category* | Citation of document, with indication, where appropriate, of the relevant passages | Relevant to claim No. |
| --- | --- | --- |
| Y | JP 45-30189 B1 (ASAHI DENKA KOGYO KK.) 30 September 1970, examples (Family: none) | 1-5 |
| Y | WO 98/44146 A1 (GREEN CROSS CORP.) 08 October 1998, example 4 & US 6309864 B1, example 4 & EP 1004673 A1 | 1-5 |
| Y | JP 50-121482 A (SANYO CHEMICAL INDUSTRIES, LTD.) 23 September 1975, example 6, table 1 (Family: none) | 1-5 |
| Y | 齋藤好廣, プルロニック系界面活性剤, 日本油化学会誌, 2000, vol. 49, no. 10, pp. 1071-1080, in particular, table 1, (SAITO, Yoshihiro, "Pluronic Surfactants", Journal of Japan Oil Chemists' Society) | 1-5 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **TADASHI BABA.** *"Kozoseibutsu" (structural biology),* 1998, vol. 5 (1), 35-39
- **NOBUYUKI ESAKI et al.** *"Seikagaku kiso no kiso"* (introduction to basic biochemistry). Kagaku-Dojin Publishing Co., Inc, March 2002, 288-289
- Baio jikken irasutoreiteddo 7 (biology experiments illustrated 7). Takayuki Mizuno, 30 October 2003, 47-69
- **BECKER ; GUARENTE.** *Methods Enzymol.,* 1991, vol. 194, 182-187
- **TAKEHIKO FUJIMOTO.** Kaimen kasseizai nyumon (Introduction to Surfactants). Sanyo Chemical Industries, Ltd, 2007, 142